# EUROPÄISCHE PATENTSCHRIFT

(11) **EP 3 697 455 B1**
(45) Veröffentlichungstag und Bekanntmachung des Hinweises auf die Patenterteilung: **12.10.2022**
(21) Anmeldenummer: 17793855.2
(22) Anmeldetag: 16.10.2017
(51) Int. Cl.: A61L 2/07, A23L 3/18, A23L 3/24, A23L 3/50, B65B 1/16, B65B 55/18, A23C 3/037, B65B 55/14

(54) **PULVERENTKEIMUNGSVERFAHREN**
POWDER STERILIZATION METHOD
PROCÉDÉ DE STÉRILISATION DE POUDRE

(43) Veröffentlichungstag der Anmeldung: 26.08.2020
(73) Patentinhaber: Fydec Holding SA, 1024 Ecublens (CH)
(72) Erfinder: DIETRICH, Yves, 1024 Ecublens (CH); DIETRICH, Frederic, 1054 Morrens (CH)
(74) Vertreter: Patent- und Rechtsanwälte Behrmann Wagner PartG mbB
(86) Internationale Anmeldenummer: PCT/EP2017/076355
(87) Internationale Veröffentlichungsnummer: WO 2019/076434

(56) Entgegenhaltungen:
- EP-A2- 0 803 203
- DE-B3-102010 054 649
- US-A- 2 303 422
- US-A1- 2005 037 119
- Josef T Hörmansperger: "Quality Preservation of Functional Food Powders by Short-Time Vacuum-Steam-Vacuum Decontamination Treatment", , 1 January 2015 (2015-01-01), XP055623833, DOI: 10.3929/ETHZ-A-010530705 Retrieved from the Internet: URL:https://www.research-collection.ethz.c h/bitstream/handle/20.500.11850/104920/eth -48121-02.pdf?sequence=2&isAllowed=y

## Beschreibung

Die Erfindung betrifft ein Pulvereinkeimungsverfahren zum Entkeimen von Pulver, insbesondere einem pharmazeutischen Wirkstoffpulver oder einem Lebensmittelpulver, wie Milchpulver, mit Fluiddampf, insbesondere Wasserdampf, wobei das Pulver in einem evakuierten, d.h. mit Unterdruck beaufschlagten bzw. auf einem Unterdruckniveau liegenden Behandlungsraum mit dem Fluiddampf beaufschlagt wird.

Des Weiteren betrifft die vorliegende Offenbarung eine Pulverentkeimungsvorrichtung zur Durchführung eines erfindungsgemäßen Pulverentkeimungsverfahrens, wobei die Vorrichtung einen, insbesondere langgestreckten, weiter bevorzugt rohrförmigen, den ebenfalls bevorzugt langgestreckten Behandlungsraum begrenzenden Behälter (Reaktor) und diesem zugeordnete Evakuierungsmittel, insbesondere umfassend eine Vakuumpumpe, zum Evakuieren des Behandlungsraums, d.h. zum Beaufschlagen des Behandlungsraums mit Unterdruck aufweist. Ferner umfasst die Pulverentkeimungsvorrichtung Fluiddampfbeaufschlagungsmittel zum Beaufschlagen des Pulvers in dem evakuierten Behandlungsraum mit dem Fluiddampf. Die Fluiddampfbeaufschlagungsmittel umfassen bevorzugt wiederum einen Dampferzeuger sowie mindestens eine Dampfleitung zum Zuführen des Fluiddampfes, insbesondere des Wasserdampfes in den Behandlungsraum des Behälters über mindestens eine Austrittsöffnung, ganz besonders bevorzugt über mindestens eine Austrittsdüse.

Die Entkeimung, d.h. Reduzierung der vorhandenen Mikroorganismen-Keimzahlen pro Materialmenge ist in der Regel eine entsprechende Behandlung von Oberflächen. Bei einer Entkeimung von Oberflächen, wie von natürlichen Oberflächen von Pflanzenteilen oder Fleischstücken oder künstlichen Oberflächen kommt es darauf an, neben einer hohen Entkeimungswirksamkeit auch die durch die Oberflächen begrenzten Materialien möglichst in ihrem Ausgangszustand zu belassen. Besondere Schwierigkeiten bereitet in der Praxis die Entkeimung von Pulver, also von Materialpartikelschüttungen.

Zur Entkeimung sind verschiedene Verfahren bekannt geworden, die sich in chemische, physikalische und thermische Verfahren unterteilen lassen. So ist es bekannt zur Keimreduzierung bei Gewürz- und Heilpflanzen chemische Verfahren, wie eine Ethylenoxid- und Ozonbehandlung einzusetzen, wobei bei ersteren gesundheitliche Bedenken bestehen. Auch ist es bekannt physikalische Methoden einzusetzen, insbesondere die Bestrahlung mit ionisierenden Strahlen, die jedoch zwischenzeitlich zur Entkeimung von Lebensmitteln verboten sind. Daneben sind auch Hochdruckentkeimungsverfahren bei sehr hohen Drücken bis 7000 bar bekannt.

Gemäß der Offenbarung der DE 198 18 224 B4 ist in dem GB-Buch "Engineering and Food", Vol. 2, Elsevier London 1984, Seite 595 ein thermisches Entkeimungsverfahren für Pulver beschrieben, bei dem überhitzter Dampf zum Einsatz kommt. Der Nachteil des bekannten Verfahrens liegt in der fehlenden Möglichkeit, durch Kondensieren des Dampfes in kurzer Zeit eine große Energiemenge auf das Material zu übertragen.

Ein entsprechend verbessertes, jedoch nicht für die Anwendung bei Pulvern geeignetes Verfahren ist in der DE 198 18 224 B4 selbst beschrieben. Kern des bekannten Verfahrens ist es, das Behandlungsgut mit Sattdampf zu beaufschlagen und anschließend das sich auf dem Behandlungsgut bildende Kondensat durch Absenken des Drucks in einem Behandlungsraum wieder zu verdampfen und damit die Substanzen zu trocknen. Bei Pulvern würde das bekannte Verfahren zu Verklumpungen führen.

Auch in der WO 2009/003546 A1 ist ein Verfahren zur Oberflächenentkeimung von Lebensmittelstücken beschrieben, die hierzu unter Rührkrafteinwirkung mit Sattdampf beaufschlagt werden, der dann auf den Lebensmittelproduktstücken kondensiert, wobei das Kondensat durch entsprechende Druckabsenkung wieder verdampft. Für Pulver ist das bekannte Verfahren aufgrund von dessen Verklumpungsneigung nicht geeignet.

Die DE 10 2010 054 649 B3 beschreibt ein Pulverdosierverfarhen und eine Pulverdosiervorrichtung. Die US 2,303,422 A handelt von einem Verfahren zum Abfüllen von Lebensmitteln. Die US 2005/0037119 A1 offenbart die Auflösung eines Rohmaterials, die Verschmelzung eines Rohmaterials oder die Behandlung zur Verbesserung der Fließfähigkeit eines Rohmaterials unter Einwirkung von Fluiddampf.

Die EP 0 803 203 A beschreibt eine Vorrichtung, in der Pulver zunächst als Haufwerk an das obere Ende einer Fallstrecke gefördert wird und dann die Fallstrecke durchquert, bevor es erneut von einer Hebevorrichtung zum oberen Ende der Fallstrecke transportiert wird. Dabei ist vorgesehen, dass zunächst, vor Beginn der Pulverbehandlung, ein Unterdurck erzeugt wird, um trockene Atmosphäre möglichst vollständig zu entfernen. Anschließend und ebenfalls noch vor Beginn der Pulverbehandlung ist dann die Erzeugung einer feuchten oder Fluiddampf aufweisenden Überdruckatmosphäre vorgesehen, in der dann die Pulverbehandlung stattfindet.

Die Dissertation "Quality preservation of functional food powders by shorttime vacuum-steam-vacuum decontamination treatment" von Josef T. Hörmansperger beschreibt bereits einen Fallturm zur Dekontamination von Pulver, in dem zunächst ein Unterdruck oder Vakuum angelegt oder erzegt wird, bevor das Pulver eine Fallstrecke durchläuft, in der es mit Fluiddampf beaufschlagt wird. In einem anschließenden Schritt wird das Pulver, welches sich im Bodenbereich des Fallturms gesammelt hat, erneut einem Vakuum oder Unterdruck ausgesetzt.

Ausgehend von dem vorgenannten Stand der Technik liegt der Erfindung die Aufgabe zugrunde, ein verbessertes Verfahren zur Entkeimung von Pulver anzugeben, mit dem das Pulver möglichst schonend entkeimbar ist. Diese Aufgabe wird hinsichtlich des Pulverentkeimungsverfahrens mit den Merkmalen des Anspruchs 1 gelöst.

Ganz besonders bevorzugt ist es als Fluiddampf Wasserdampf einzusetzen, wobei hier grundsätzlich auch andere hiervon abweichende Fluide zum Einsatz kommen können.

In der vorliegenden Offenbarung liegt der Schwerpunkt auf der Erläuterung des erfindungsgemäßen Verfahrens. Auch wenn dies nicht in jedem Fall ausdrücklich erwähnt ist, umfasst die Vorrichtung jeweils entsprechende Funktionsmittel zur Durchführung entsprechender Verfahrensschritte. So umfasst die Vorrichtung beispielsweise für den Fall der Beschreibung einer bevorzugter Erwärmung bzw. Erhitzung des Behandlungsraums entsprechende Heizmittel.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Zur Vermeidung von Wiederholungen sollen vorrichtungsgemäß offenbarte Merkmale auch als verfahrensgemäß offenbart sein und beanspruchbar sein.

Zunächst liegt der Erfindung die Erkenntnis zugrunde, das eine schonende Entkeimung von Pulver eine möglichst kurze Beaufschlagung mit hohen Temperaturen erfordert, wie dies mit der Beaufschlagung von Fluiddampf, insbesondere Sattdampf realisierbar ist, um somit - je nach Pulverbeschaffenheit - Thiaminverluste, Lipidoxidationen oder Maillard-Reaktionen, etc. zu verhindern oder auf ein Minimum zu begrenzen.

Um den Einsatz einer Fluiddampfentkeimung für Pulver zu ermöglichen und dabei Verklumpungen zu vermeiden, ist erfindungsgemäß vorgesehen, das Pulver nicht in der Form eines Haufwerks oder während einer mechanischen Durchmischung mit Fluiddampf zu beaufschlagen sondern während eines freien Falls, d.h. während das Pulver entlang einer erfindungsgemäß vorgesehenen, sich entlang der Vertikalen erstreckenden Fallstrecke rieselt bzw. fällt. Ferner hat die Erfindung erkannt, dass die Effektivität der Entkeimung deutlich verbessert werden kann, wenn die Dampfbeaufschlagung während des freien Falls im Unterdruck, d.h. unterhalb vom Normaldruck stattfindet - anders ausgedrückt rieselt das Pulver, insbesondere in Form einer Pulverwolke in einen evakuierten Behandlungsraum entlang der Fallstrecke, wo dann die Fluiddampfbeaufschlagung erfolgt. Durch Evakuieren des Behandlungsraums wird nämlich gewährleistet, dass ein ansonsten das unmittelbare Auftreffen des Fluiddampfs auf das Pulver, d.h. die Materialpartikel verhindernde Luftbarriere bzw. Luftpolster nicht vorhanden oder zumindest reduziert ist bzw. sich nicht ausbilden kann, wodurch ein unmittelbares Auftreffen des Fluiddampfes auf die Pulverpartikel bzw. ein inniger Kontakt des Fluiddampfes mit dem Pulver gewährleistet wird. Der Fluiddampf wird somit nicht um die Pulverpartikel herum abgeleitet, sondern trifft diese unmittelbar.

Das erfindungsgemäße Entkeimungsverfahren und die zur Durchführung des Verfahrens geeignete Pulverentkeimungsvorrichtung sehen also vor das Pulver in einem evakuierten, d.h. mit einem Druck unter Normaldruck beaufschlagten bzw. aufweisenden Behandlungsraum während eines freien Falls, während eines Rieselns entlang einer Fallstrecke mit dem Fluiddampf, insbesondere im Gegenstrom, zu beaufschlagen. Die Vorrichtung umfasst zur Realisierung der Fallstrecke einen die Fallstrecke aufweisenden bzw. beinhaltenden, insbesondere entlang einer Vertikalen langgestreckten Behandlungsraum, der mit entsprechenden Evakuierungsmitteln, insbesondere einer Vakuumpumpe evakuierbar ist und in dem Pulver während des freien Falls entlang der Fallstrecke mit dem Fluiddampf beaufschlagbar ist, wozu vorzugsweise entsprechend mindestens eine Fluiddampfauslassöffnung entlang der und/oder unterhalb der Fallstrecke angeordnet ist, insbesondere derart, dass Fluiddampf, vorzugsweise schräg, nach oben ausgeblasen wird, um einen Gegenstrom zu dem nach unten rieselenden Pulver zu erzeugen.

Im Hinblick auf die konkrete Ausgestaltung des Pulvers gibt es die unterschiedlichsten Möglichkeiten. So kann es sich beispielsweise um ein Lebensmittelpulver, insbesondere Milchpulver handeln, welches generell leicht zu Verklumpungen neigt. Auch kann es sich bei dem Pulver um einen pharmazeutischen Wirkstoff handeln, etc. Bevorzugt ist eine mittlere Partikelgröße der Pulverpartikel, insbesondere ein mittlerer Partikeldurchmesser X_{50,3} der Pulverpartikel, aus einem Wertebereich zwischen 0,1 µm und 200µm, ganz besonders bevorzugt zwischen 1µm und 100µm gewählt.

Ganz besonders bevorzugt ist eine Weiterbildung des erfindungsgemäßen Verfahrens bei der der Fluiddampf, insbesondere Sattdampf auf einer Pulveroberfläche, d.h. der Oberfläche der Pulverpartikel, kondensiert, woraus ein optimierter Wärmeübergang resultiert, der es ermöglicht, die hohe Temperaturbeaufschlagung der Pulverpartikel auf eine sehr kurze Zeitspanne zu reduzieren, wobei zur Vermeidung eines Verklumpens des Pulvers im Falle einer Kondensation auf der Oberfläche der Pulverpartikel weiter vorgesehen ist, das Pulver vor dem Abführen aus dem Behandlungsraum (wieder) zu trocknen, und zwar durch Verdampfen von Kondensat von der Pulveroberfläche, was durch entsprechende Temperatur- und insbesondere Unterdruckbeaufschlagung bzw. -steuerung erzielbar ist. Ganz besonders zweckmäßig ist es, die Fallstrecke für den Trocknungsvorgang zu nutzen, derart, dass an einen Fluiddampfbeaufschlagungsabschnitt der Fallstrecke ein Trocknungsabschnitt der Fallstrecke anschließt, in der das Kondensat, insbesondere durch entsprechende Unterdruckbeaufschlagung der Fallstrecke bzw. des die Fallstrecke beinhaltenden Behandlungsraums zumindest teilweise, vorzugsweise größtenteils, ganz besonders bevorzugt vollständig wieder von der Pulveroberfläche verdampft und im Ergebnis entkeimtes und bereits wieder getrocknetes Pulver am unteren Ende der Fallstrecke ankommt und dann, insbesondere in Form eines sich bildenden Haufwerks, wieder aus dem Behandlungsraum abführbar ist bzw. abgeführt wird.

Ganz besonders bevorzugt ist eine Ausführungsvariante des erfindungsgemäßen Verfahrens, bei denen das zu entkeimende Pulver bei auf einem ersten Unterdruckniveau, bevorzugt zwischen 0,1mbar (oder kleiner) und 300mbar absolut, ganz besonders bevorzugt zwischen 0,1mbar und 100mbar absolut befindlichem Behandlungsraum diesem zugeführt wird (wobei als untere Grenze des ersten Unterdruckniveaus grundsätzlich auch ein Druck von über 0,1mbar absolut, beispielsweise von 0,5mbar oder 0,1bar absolut realisierbar bzw. denkbar ist.) und während des freien Falls entlang der Fallstrecke mit dem Fluiddampf beaufschlagt wird, wobei, insbesondere durch die Zuführung bzw. Einleitung des Fluiddampfes, das Druckniveau im Behandlungsraum auf ein verglichen mit dem ersten Unterdruckniveau erhöhtes zweites Druckniveau (entweder ein Unterdruckniveau, ein Normaldruckniveau oder ein Überdruckniveau), bevorzugt mit einem Behandlungsraumdruck von über 0,5bar absolut, bevorzugt auf einen Druck aus einem Wertebereich zwischen 0,7bar und 4bar absolut, insbesondere zwischen 1bar und 3bar absolut, ansteigt, und dass das Druckniveau vor dem Abführen des

Pulvers aus dem Behandlungsraum, insbesondere noch während des freien Falls des Pulvers entlang der Fallstrecke zum Trocknen des Pulvers abgesenkt wird, insbesondere auf ein (drittes) Unterdruckniveau, bevorzugt aus einem Wertebereich zwischen 0,1mbar (oder kleiner) und 600mbar absolut, ganz besonders bevorzugt zwischen 0,1mbar und 300mbar absolut. Erfindungsgemäß vorgesehen ist, dass die Evakuierungsmittel zur Bereitstellung des Unterdrucks (insbesondere des ersten Unterdruckniveaus und des dritten Unterdruckniveaus) im Behandlungsraum dauerhaft, d.h. kontinuierlich betrieben werden, sodass ständig ein Saugdruck anliegt, und der Druck nach dem Zuführen des Pulvers während des freien Falls durch Fluiddampfbeaufschlagung ansteigt, wobei, wie erwähnt, gerade auch während dieser Fluiddampfbeaufschlagung mittels der Evakuierungsmittel, insbesondere einer Vakuumpumpe aus dem Behandlungsraum abgesaugt wird. Ganz besonders bevorzugt ist es, wenn das dritte Unterdruckniveau, auf das der Absolutdruck im Behandlungsraum (Reaktorraum) noch während des freien Falls des Pulvers entlang der Fallstrecke zum Trocknen des Pulvers abgesenkt wird dem ersten (absoluten) Unterdruckniveau entspricht. Für den Fall, dass die Fallzeit bzw. Fallstrecke zum Absenken des Drucks auf das erste Unterdruckniveau noch während des freien Falls der Pulverpartikel nicht ausreichen sollte, kann das dritte Unterdruckniveau auch oberhalb des ersten Unterdruckniveaus liegen, wobei es bevorzugt ist, wenn das Behandlungsdruckniveau vor dem erneuten Aufgeben bzw. Zuführen einer zu entkeimenden und dann wieder zu trocknenden Pulverportion (wieder) auf das erste Unterdruckniveau abgesenkt wird. Bevorzugt ist es, wenn noch während des freien Falls ein drittes Unterdruckniveau von mindestens 600mbar oder kleiner erreicht wird. Bevorzugt wird vorstehender Prozess nacheinander immer wieder für jede neue Pulverportion wiederholt, sodass im Ergebnis aufgrund der kurzen Behandlungszeiten eine quasikontinuierliche Entkeimung und Trocknung resultiert.

Ganz besonders bevorzugt wird das Verfahren so ausgestaltet, dass nicht dauerhaft Fluiddampf in die Fallstrecke eingespeist wird sondern nur während eines Zeitabschnitts der Pulverbehandlung. Besonders bevorzugt ist die Fluiddampfeinspeisung während der Zuführung von zu entkeimendem Pulver in den Behälter und/oder während der Trocknungsphase, insbesondere während des Fallens des Pulvers durch den Trocknungsabschnitt unterbrochen bzw. findet nicht statt.

Um die Zeit zum Aufbau eines Unterdruckniveaus zu reduzieren ist es bevorzugt, wenn zu entkeimendes Pulver über eine Einlass-Unterdruckschleuse, insbesondere eine Flügelradschleuse, in den bereits evakuierten, d.h. auf einem, insbesondere dem ersten Unterdruckniveau liegenden Behandlungsraum zugeführt wird und nach dem freien Fall entlang der Fallstrecke und nach der währenddessen durchgeführten Fluiddampfbeaufschlagung (hierdurch entkeimtes und bevorzugt bereits wieder wie zuvor beschrieben getrocknetes) Pulver über eine Auslass-Unterdruckschleuse, insbesondere eine Flügelradschleuse, aus dem Behandlungsraum abgeführt ist.

Ganz besonders bevorzugt ist es, wenn zu entkeimendes Pulver portionsweise zugeführt und/oder wieder portionsweise aus dem Behandlungsraum abgeführt wird, was besonders einfach mittels Zellen einer Flügelradschleuse realisierbar ist. Eine portionsweise bzw. batchweise Zuführung ermöglicht eine besonders einfach getaktete Vorgehensweise, im Rahmen derer jede Pulverportion für sich entlang der Fallstrecke fällt, für eine begrenzte Zeitspanne mit Fluiddampf beaufschlagt und bevorzugt dann während des freien Falls wieder getrocknet wird. Grundsätzlich ist es möglich zu entkeimendes Pulvers ausschließlich während oder ausschließlich nach dem Abführen von entkeimtem Pulver, insbesondere über die erwähnte Auslass-Unterdruckschleuse dem Behandlungsraum über die erwähnte Einlass-Unterdruckschleuse zuzuführen. Möglich ist jedoch auch eine Überschneidung dieser Vorgänge, also derart, dass zu entkeimendes Pulver bereits während und nach dem Abführvorgang zum Abführen von entkeimtem Pulver dem Behandlungsraum zugeführt wird bzw. im Rahmen der Vorrichtung zuführbar ist.

Ganz besonders bevorzugt ist in Weiterbildung der Erfindung vorgesehen, wenn als Unterdruckschleuse zur Zuführung von zu entkeimenden Pulver in den Behandlungsraum eine volumetrische Pulverdosiervorrichtung vorgesehen bzw. dem Behandlungsraum vorgeordnet ist, die beispielsweise wie in der EP 2 652 541 B1 beschrieben ausgebildet sein kann. Bevorzugt zeichnet sich eine solche Pulverdosiervorrichtung durch eine Dosierkammer aus, die mit einer Unterdruckleitung zum Beaufschlagen der Dosierkammer mit Unterdruck verbunden bzw. verbindbar ist, um dadurch zu entkeimendes Pulver aus einem Pulvervorrat ansaugen und die Dosierkammer mit zu entkeimenden Pulver befüllen zu können. Das Dosierkammervolumen (zu entkeimendes Pulvervolumen) kann dann, insbesondere durch Verbinden mit einer Druckgasleitung und/oder über einen verstellbaren Auswurfstempel, in Richtung Behandlungsraum, bevorzugt unmittelbar in den Behandlungsraum hinein entleert werden, wobei es bevorzugt ist, wenn die Dosierkammer während des Füllvorgangs der Dosierkammer durch Ansaugen von zu entkeimenden Pulver auslassseitig durch geeignete, bevorzugt unterdruckdichte, Verschlussmittel, beispielsweise mittels einer Klappe oder eines Schiebers geschlossen ist und bevorzugt dadurch vom Behandlungsraum druckmäßig entkoppelt ist. Nach Öffnen der

Verschlussmittel kann das (dosierte) Pulver aus der Dosierkammer in den Behandlungsraum gelangen und dort frei fallen. Durch das Vorsehen einer unterdruckbasierten Pulverdosiervorrichtung als Unterdruckschleuse können Druck- bzw. Unterdruckverluste, wie diese bei einer Flügelradschleuse nur schwer bzw. mit großem Aufwand vermeidbar sind, sicher vermieden werden. Darüber hinaus ist eine exakte volumetrische Dosierung von nacheinander zu entkeimenden Pulverportionen möglich. Bevorzugt zeichnet sich die Pulverdosiervorrichtung durch einen Unterdruckanschluss sowie ein Druckgasanschluss aus, über die die Dosierkammer wechselseitig mit Unterdruck und Überdruck zum Befüllen und Entleeren der Unterdruckkammer beaufschlagbar ist. Bevorzugt ist das Dosierkammervolumen einstellbar, insbesondere durch ein axiales Verstellen eines fakultativen Stempels, der zusätzlich oder alternativ zu einer Druckbeaufschlagung zur Entleerung des zu entkeimenden Pulvers in den Behandlungsraum hinein verstellbar angeordnet sein kann bzw. bevorzugt ist.

Ganz besonders bevorzugt ist es, wenn, insbesondere durch entsprechende Ausgestaltung der Fluiddampfbeaufschlagungsmittel, insbesondere eines Dampferzeugers Fluiddampf mit einem verglichen mit dem Druckniveau im Behandlungsraum zum Zeitpunkt eines Startes (Beginns) der Fluiddampfbeaufschlagung erhöhten Druck, insbesondere aus einem Wertebereich zwischen 1,1bar und 6bar (absolut), weiter bevorzugt zwischen 1,1bar und 4bar (absolut) in den Behandlungsraum eintritt bzw. dem Behandlungsraum zuführbar ist.

Wie erwähnt ist es besonders zweckmäßig, wenn sich Fluiddampf und Pulver im Gegenstrom bewegen, wozu es notwendig ist, den Fluiddampf mit zumindest einer Richtungskomponente nach oben, d.h. in vertikaler Richtung ausströmen zu lassen. Grundsätzlich ist es denkbar, den Fluiddampf exakt vertikal einzuspeisen - besonders bevorzugt ist jedoch eine Ausführungsform, bei der ein Winkel zwischen einer Hauptausströmungsrichtung des Fluiddampfes aus mindestens einer entsprechenden Auslassöffnung, insbesondere einer Auslassdüse zur Vertikalen zwischen 1° und 60°, ganz besonders bevorzugt zwischen 5° und 45°, noch weiter bevorzugt zwischen 10° und 30° beträgt. Eine derartige Verfahrensführung bzw. Vorrichtungsausgestaltung lässt sich besonders geeignet durch das Vorsehen einer Ringauslass-, insbesondere Ringdüsenanordnung realisieren.

Besonders zweckmäßig ist es, wenn der Fluiddampf durch das entsprechende Vorsehen verschiedener Auslassöffnungen, insbesondere Auslassdüsen an mehreren vertikal beabstandeten Stellen entlang der Fallstrecke und/oder an mehreren in Umfangsrichtung (um eine Fallachse herum) beabstandeten Stellen in den Behandlungsraum eintritt bzw. einspeisbar ist.

Ganz besonders bevorzugt ist es mehrere entlang der Vertikalen übereinander angeordnete Ringdüsenanordnungen jeweils aufweisend eine Mehrzahl von in Umfangsrichtung beabstandeten Auslassdüsen für Fluiddampf vorzusehen. In einer einfachen Ausführungsform ist es auch möglich, eine einzige Ringdüsenanordnung im Bereich der Fallstrecke oder darunter anzuordnen, jedenfalls derart, dass das Pulver während des freien Falls mit dem Fluiddampf beaufschlagbar ist.

Besonders zweckmäßig ist es, insbesondere durch entsprechende Ausgestaltung und/oder einen entsprechenden Betrieb der Fluiddampfbeaufschlagungsmittel, insbesondere eines Fluiddampferzeugers, wenn der in dem Behandlungsraum eingespeiste Fluiddampf (beim Dampfeintritt) gesättigt oder alternativ, insbesondere leicht, überhitzt ist, wobei es besonders bevorzugt ist, wenn dieser bei Eintritt in den Behandlungsraum eine Temperatur aus einem Wertebereich zwischen 101°C und 200°C oder höher, ganz besonders bevorzugt zwischen 110°C und 160°C aufweist.

Wie erläutert ist es möglich, dass es sich bei dem eingeschweißten Fluiddampf (beim Dampfeintritt) um gesättigten Dampf handelt - bevorzugt ist jedoch eine Variante, bei der der Dampf beim Dampfeintritt überhitzt ist, um somit die Kondensatmenge zu reduzieren - der Dampf geht dann in der Unterdruckatmosphäre ohnehin in Sättigung. Bevorzugt sind die Dampfparameter (Druck, Temperatur, Feuchtigkeitsgehalt) so gewählt, dass eine Kondensation des Dampfes in der mindestens einen Dampfleitung zwischen einen Dampferzeuger und den Austrittsöffnungen, insbesondere Düsen, verhindert wird, so dass insbesondere kein Kondensat in den Behandlungsraum eingeschossen werden kann. Da der Dampf beim Einschießen in den Behandlungsraum aufgrund der Expansion im Unterdruck einen hohen Energieanteil verliert bzw. abgibt, jedoch bevorzugt ein hoher Energieanteil für die Inaktivierung oder Zerstörung von Keimen bzw. Mikroorganismen zur Verfügung steht, ist eine Überhitzung des Dampfes (beim Dampfeintritt) sinnvoll bzw. bevorzugt.

Um eine Dampfkondensation an der Behälterwand zu verhindern und den bevorzugten, fakultativen Trocknungsprozess zu beschleunigen hat es sich als vorteilhaft herausgestellt, wenn der Behandlungsraum auf eine Temperatur aus einem Wertebereich zwischen 150°C und 400°C, bevorzugt zwischen 180°C und 250°C erwärmt wird bzw. während des Entkeimungsprozesses erwärmt ist. Hierzu kann beispielsweise eine Wandheizung eingesetzt werden, insbesondere eine ÖI- oder Dampfheizung, um Temperaturen oberhalb von 100°C einfach einstellen bzw. halten zu können. Bevorzugt ist die Heizung leistungsmäßig so dimensioniert, dass die Behandlungsraumtemperatur in erster Näherung der Vorlauftemperatur der Heizung entspricht.

Grundsätzlich ist es möglich, dass das zugeführte Pulver unmittelbar aus einer Zuführposition heraus entlang der Fallstrecke ohne weitere Maßnahmen fällt, insbesondere nach Passieren der Einlass-Unterdruckschleuse. Um eine Pulverwolkenbildung zu verbessern, sprich das Pulver besser, insbesondere gleichmäßiger zu verteilen und ggf. den Rieselvorgang (zeitlich) zu entzerren, sodass Pulverpartikel aufgelockert über eine Zeitspanne verteilt nacheinander entlang der Fallstrecke fallen um somit sicherzustellen, dass ein Großteil, insbesondere sämtliche Partikel jeweils von Fluiddampf getroffen bzw. mit diesem beaufschlagt werden, ist in Weiterbildung der Erfindung vorgesehen entsprechende Funktionsmittel vorzusehen, und entweder in die Zuführung zu integrieren oder bevorzugt dieser nachzuordnen. Ganz besonders bevorzugt erfolgt das Verbessern der Pulverwolkenbildung durch entsprechende Funktionsmittel als separater Schritt nach dem Zuführen des Pulvers in den Behandlungsraum. Die Funktionsmittel sind dabei so ausgebildet, dass mit diesen eine Pulverwolkenbildung verbesserbar ist, insbesondere indem sie das zugeführte Pulver, insbesondere eine zugeführte Pulverportion vereinzeln oder verwirbeln und/oder auflockern. So ist es beispielsweise möglich mechanische Verteil- und/oder Auflockerungsmittel vorzusehen, insbesondere umfassend Vibrationsmittel, mit denen beispielsweise ein Sieb zur Auflockerung und Verteilung von Pulver vibrierbar ist, wobei das Pulver dieses vibrierende Element, insbesondere Sieb vor oder im oberen Bereich der Fallstrecke durchlaufen muss. Auch ist es denkbar strömungsmechanische Verteil- und/oder Auflockerungsmittel einzusetzen, beispielsweise in Form von Gasdüsen, die das zugeführte Pulver, insbesondere eine Pulverportion auflocken bzw. verwirbeln.

Ganz besonders bevorzugt ist es, wenn das Pulver nur während einer Fallzeit zwischen 0,05s und 2s, insbesondere zwischen 0,1s und 1,5s, noch weiter bevorzugt zwischen 0,1s und 0,5s mit Fluiddampf beaufschlagt wird/beaufschlagbar ist.

Ganz besonders bevorzugt ist es, wenn das Pulver, insbesondere ein Lebensmittelpulver, durch den Trocknungsvorgang bis zu einem maximalen Restfeuchtegehalt von 15 Gew.-%, ganz besonders bevorzugt 5 Gew.-%, noch weiter bevorzugt 4 Gew.-% getrocknet wird. Für pharmazeutische Anwendungen hat sich als vorteilhaft herausgestellt, wenn das, insbesondere pharmazeutische, Pulver ganz besonders bevorzugt ein pharmazeutischer Wirkstoff auf einen maximalen Restfeuchtegehalt aus einem Wertebereich zwischen 0 Gew.-% und 5 Gew.-%, ganz besonders bevorzugt zwischen 0,01 Gew.-% und 5 Gew.-%, noch weiter bevorzugt zwischen 0,1 Gew.-% und 3 Gew.-%, ganz besonders bevorzugt zwischen 0,1 Gew.-% und 1 Gew.-% getrocknet wird.

Bevorzugt ist es, wenn die Axialerstreckung, d.h. die Länge der Fallstrecke entlang der Vertikalen aus einem Wertebereich zwischen 2m und 30m, insbesondere zwischen 4m und 15m gewählt ist

Auch ist es zweckmäßig, den Innendurchmesser des Behandlungsraums aus einem Wertebereich zwischen 50mm und 2000mm, ganz besonders bevorzugt zwischen 80mm und 1000mm zu wählen.

Grundsätzlich ist es möglich und bevorzugt, den Behandlungsraum im Inneren, d.h. in dessen radialem Zentrum leer bzw. frei zu gestalten, insbesondere wenn dieser eine hohlzylindrische, ganz besonders bevorzugt hohlkreiszylindrische Kontur aufweist. Zur Realisierung großer Massedurchsätze ist es alternativ auch möglich und bevorzugt, den Behandlungsraum als Ringraum um ein, insbesondere ausgefülltes, Zentrum (Zentralabschnitt) herum auszugestalten, wobei dann bevorzugt die Dampfeinbringung nicht nur aus einer Richtung, insbesondere nicht nur von radial außen erfolgt, sondern zusätzlich vom Zentral- bzw. Innenbereich her. Mit anderen Worten ist also in Weiterbildung der Erfindung vorgesehen, einen in einer Querschnittsansicht ringförmig, insbesondere kreisringförmig konturierten Behandlungsraum zu realisieren und Dampfaustrittsöffnungen, insbesondere Düsen, ganz besonders bevorzugt Ringdüsen sowohl im Bereich einer den Behandlungsraum radial außen begrenzenden Behälter-Außenwand (auf deren Innenseite), als auch im Bereich einer den Behandlungsraum radial innen begrenzenden zentrischen bzw. inneren Behälterwand (auf deren Außenseite) anzuordnen. Hierdurch lassen sich auch große Raumvolumina besonders gleichmäßig mit Fluiddampf beaufschlagen, um somit eine gute Durchmischung mit dem Pulver bzw. einer Pulverportion zu gewährleisten.

Als besonderes zweckmäßig hat sich ein Verhältnis zwischen einer Fallstreckenlänge und einem Innendurchmesser aus einem Wertebereich zwischen 20 und 150, ganz besonders bevorzugt zwischen 40 und 100 herausgestellt, wobei der Innendurchmesser gemessen wird von zwei diametral einander gegenüberliegenden Stellen einer dem Behandlungsraum radial außen begrenzenden Behälter-Außenwand, wobei bevorzugt bei größeren Innendurchmessern, insbesondere von über 500mm der Behandlungsraum innen von einem, insbesondere kreiszylindrischen, ganz besonders bevorzugt axial durchgehenden oder alternativ nur axial abschnittsweise vorgesehenen Zentralabschnitt begrenzt wird, wobei es besonders bevorzugt ist, wenn die Dampfzuführung in den dann ringförmig konturierten Behandlungsraum sowohl von radial außen als auch von radial innen erfolgt.

Ganz besonders bevorzugt ist eine Ausführungsform des erfindungsgemäßen Verfahrens,
bei der die Vorrichtung eine Verpackungseinrichtung, zum insbesondere sterilen, Verpacken von entkeimten Pulver in Endverpackungen, insbesondere in Beutelverpackungen aufweist. Dabei ist es besonders zweckmäßig, wenn die Verpackungseinrichtung derart angeordnet ist, dass diese dem Behandlungsraum nachgeordnet ist, insbesondere entlang der Vertikalen unterhalb des Behandlungsraums und/oder derart, dass entkeimtes Pulver schwerkraftbedingt in eine Endverpackung, bevorzugt fallend oder rutschend, transferierbar ist.

Für den bevorzugten Fall des Vorsehens mehrerer Behandlungsräume bzw. Behälter ist es bevorzugt, wenn deren Auslässe zu einem gemeinsamen Speicher oder zu einer gemeinsamen Verpackungseinrichtung geführt sind, sodass in mindestens zwei unterschiedlichen Behandlungsräumen entkeimtes und bevorzugt getrocknetes Pulver einer gemeinsamen Verpackungseinrichtung zuführbar sind, insbesondere schwerkraftbedingt, sodass Pulver aus mindestens zwei Behandlungsräumen einer gemeinsamen Verpackungseinrichtung und damit insbesondere einer gemeinsamen Endverpackung zuführbar sind.

Ebenfalls beschrieben wird ein System, umfassend mindestens zwei Vorrichtungen zur Durchführung des erfindungsgemäßen Verfahrens, wobei das entkeimte Pulver aus diesen Vorrichtungen zusammenführbar ist, hin zu einer gemeinsamen Verpackungseinrichtung und/oder hin zu einem gemeinsamen Vorratsbehältnis. Dem System liegt ein Modulgedanke zugrunde, da es technisch überraschend einfacher realisierbar ist, größere Mengen an zu entkeimenden Pulver zu erzeugen durch das Vorsehen einer Mehrzahl von, bevorzugt identischen, kleineren Vorrichtungen als mit einer einzigen, größer dimensionierten Vorrichtung. Dabei ist es von besonderem Vorteil, wenn Pulvermassenströme von zwei Vorrichtungen
bzw. zwei Behandlungsräumen zusammengeführt und bevorzugt dadurch durchmischt werden. Bevorzugt werden Pulvermassen von mindestens zwei Behandlungsräumen zusammengeführt und einem gemeinsamen Vorratsbehälter (Speicher) zugeführt oder gemeinsam verpackt, insbesondere mittels einer gemeinsamen Verpackungseinrichtung, wobei es besonders zweckmäßig ist, wenn die Zuführung zu dieser Verpackungseinrichtung schwerkraftbedingt erfolgt, die Verpackungseinrichtung entlang der Vertikalen also unterhalb der Vorrichtungen angeordnet ist.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: eine stark schematisierte Darstellung einer Entkeimungsvorrichtung zur Durchführung eines erfindungsgemäßen Verfahrens,
- Fig. 2: ein Diagramm, bei dem der Absolutdruck in einem Behandlungsraum der Entkeimungsvorrichtung über die Zeit bzw. während eines Entkeimungs- und Trocknungsvorgangs aufgetragen ist,
- Fig. 3: eine alternative Ausführungsform einer Entkeimungsvorrichtung mit einer im Querschnitt ringförmig konturierten Behandlungsraum in den von radial innen, sowie von radial außen Dampf zur Entkeimung des Pulvers während des freien Falls einspeisbar ist bzw. eingespeist wird,
- Fig. 4: eine Ausführungsform eines Systems mit vier parallel betriebenen Entkeimungsvorrichtungen, denen jeweils zu entkeimendes Pulver portionsweise volumendosiert über die Unterdruckschleuse zuführbar ist.
- Fig. 5a: ein System, umfassend vier Entkeimungsvorrichtungen, die auslassseitig hin zu einer gemeinsamen Verpackungseinrichtung verbunden sind, und in die, analog zur Entkeimungsvorrichtung gemäß Fig. 3 und Fig. 4 eine Zudosierung von zu entkeimenden Pulverportionen nicht über eine Flügelradschleuse, sondern über volumetrische, unterdruckbasierte Volumendosiereinrichtungen erfolgt, und
- Fig. 5b: eine vakuumbasierte Volumendosiereinrichtung als Vakuumschleuse, die bei dem in Fig. 5a sowie Fig. 4 gezeigten System zum Einsatz kommt.

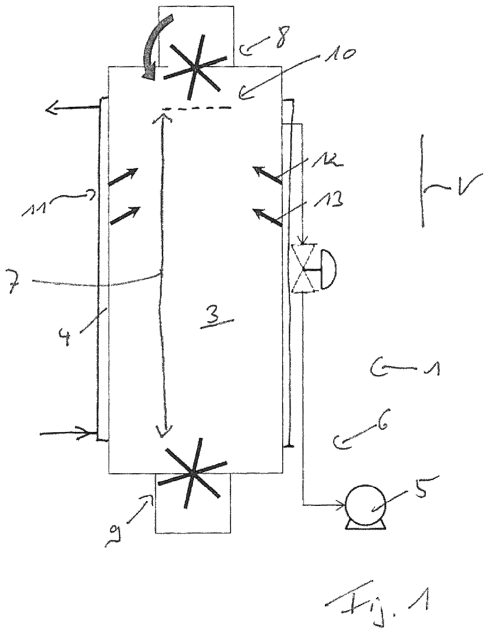

In Fig. 1 ist eine Entkeimungs-Vorrichtung 1 gezeigt. Diese umfasst einen entlang der Vertikalen langgestreckten Behälter 2, beispielsweise aus Edelstahl, der einen ebenfalls langgestreckten Behandlungsraum 3 (Reaktorraum) begrenzt. Aus der schematischen Darstellung ist das realistische Durchmesserlängenverhältnis nicht ableitbar - tatsächlich weist der Behandlungsraum 3 einen wesentlich geringeren Durchmesser als dessen Längs- bzw. Höhenerstreckung auf.

Der Behälter 2 bzw. der Behandlungsraum 3 ist über vorliegend eine als Ölheizung ausgebildete Wandheizung umfassende Heizmittel 4 erwärmbar, beispielhaft auf eine Temperatur von 300°C. Die Pfeile im linken Zeichnungsabschnitt stellen den Vorlauf und den Rücklauf dar. Der Behandlungsraum 3 ist mit einer Vakuumpumpe 5 umfassenden Evakuierungsmitteln 6 evakuierbar, d.h. auf ein Unterdruckniveau bringbar.

Der Behandlungsraum 3 umfasst eine sich entlang der Vertikalen V erstreckende Fallstrecke 7, entlang derer über eine beispielhaft als Flügelradschleuse ausgebildete Einlass-Unterdruckschleuse 8 zugeführtes Pulver entlang der Vertikalen V frei innerhalb des evakuierten Behandlungsraums 3 nach unten fallen kann, hin zu einer hier beispielhaft ebenfalls als Flügelradschleuse ausgebildeten AuslassUnterdruckschleuse 9. Bevorzugt wird anstelle einer Flügelradschleuse als Einlass-Unterdruckschleuse 8 eine, bevorzugt vakuumbasierte Pulverdosiereinrichtung zur Zudosierung von nacheinander zu entkeimenden Pulverportionen eingesetzt.

In dem gezeigten Ausführungsbeispiel sind der Einlass-Unterdruckschleuse 8 fakultative Verteilmittel 10 nachgeordnet, um eine Pulverwolkenbildung bzw. Auflockerung des zugeführten Pulvers zu verbessern. In dem gezeigten Ausführungsbeispiel umfassen die Verteilmittel 10 ein vibrierendes Gitter.

Die Fallstrecke 7 ist unterteilt in einen oberen Fluiddampfbeaufschlagungsabschnitt, in welchem das frei fallende Pulver, insbesondere in Form einer Pulverwolke, mit Fluiddampf beaufschlagbar ist. Hierzu sind stark schematisiert und nur ausschnittsweise dargestellte Fluiddampfbeaufschlagungsmittel 11 vorgesehen. Diese umfassen in dem konkreten Ausführungsbeispiel zwei entlang der Vertikalen V beabstandete Ringdüsenanordnungen 12, 13, die über eine entsprechende Dampfleitung mit einem Dampferzeuger zur Erzeugung von Sattdampf verbunden sind. An den Fluiddampfbeaufschlagungsabschnitt schließt darunter ein Trocknungsabschnitt an, in welchem das zuvor fluiddampfbeaufschlagte Pulver getrocknet wird bzw. trockenbar ist, indem das sich durch die Fluiddampfbeaufschlagung auf der Pulveroberfläche gebildete Kondensat wieder verdampft. Dies wird erreicht durch eine entsprechende Unterdruckatmosphäre während des freien Falls durch den Trocknungsabschnitt.

Im unteren Bereich angelangt wird dann das entkeimte und getrocknete Pulver über die Auslass-Unterdruckschleuse 9 aus dem auf einem Unterdruckniveau liegenden Behandlungsraum 3 ausgeschleust.

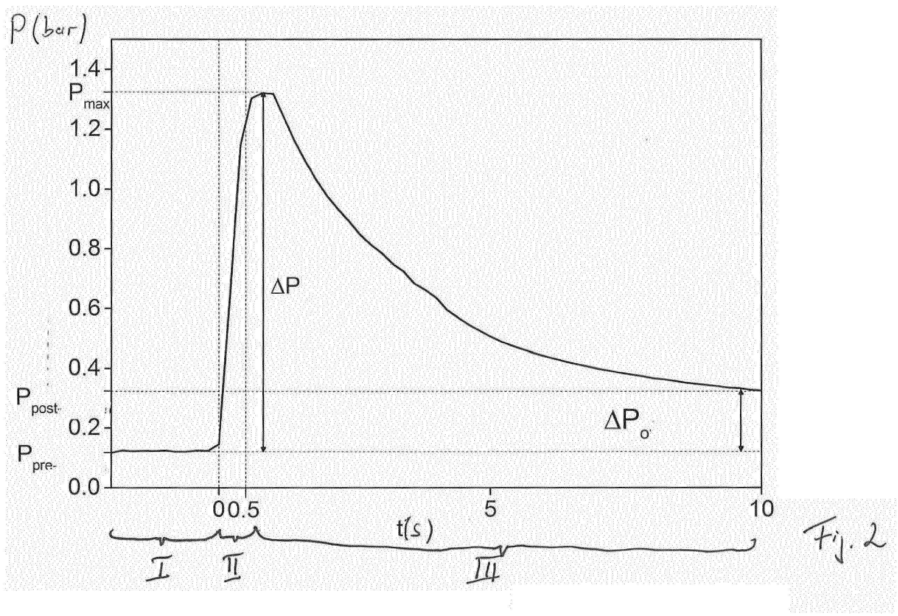

Aus Fig. 1 ist zu entnehmen, dass bei der vorliegenden Ausführungsform Pulver portionsweise zugeführt und behandelt wird, derart, dass eine zugeführte, zu entkeimende Pulverportion fakultativ mittels der Verteilmittel 10 aufgelockert bzw. verteilt wird und dann in dem evakuierten Behandlungsraum 3 nach unten entlang der Fallstrecke 7 fällt und dabei zunächst mit Fluiddampf, vorliegend Wasserdampf beaufschlagt wird. Das Pulver rieselt mit dem darauf kondensierten Kondensat weiter nach unten und wird währenddessen getrocknet. Wie später noch anhand von Fig. 2 erläutert werden wird steigt durch die Fluiddampfbeaufschlagung das Druckniveau im Behälter 2 ausgehend von einem ersten Unterdruckniveau auf ein zweites Druckniveau, beispielsweise auf oder sogar über Normaldruck (alternativ auf ein
höheres Unterdruckniveau), wobei durch die Evakuierungsmittel 6 gewährleistet wird, dass zumindest während eines Zeitabschnitts des freien Falls entlang des Trocknungsabschnittes der Fallstrecke wieder ein ausreichendes Unterdruckniveau erreicht wird, um das Kondensat zu verdampfen. Spätestens gegen Ende des Trocknungsprozesses wird ein drittes Unterdruckniveau erreicht.

In Fig. 2 ist nun ein möglicher Druckverlauf im Behandlungsraum während eines kombinierten Entkeimungs- und Trocknungsprozesses für eine jeweilige Pulverportion dargestellt bzw. über die Zeit aufgetragen. Dieser Vorgang wiederholt sich dann über die Zeit für jede neu in den Behandlungsraum 3 zugeführte Pulverportion.

Zu erkennen ist, dass während eines ersten Zeitabschnittes I, bei welchem ein erstes Unterdruckniveau mit einem Druck Pₚᵣₑ von vorliegend etwa 0,12 bar absolut herrscht, eine Pulverportion in den Behandlungsraum zugeführt, gegebenenfalls aufgelockert wird und dann jedenfalls zu fallen beginnt entlang der Fallstrecke. Während der Zeitspanne II von hier vorliegend 0,2s wird das Pulver während seines freien Falls entlang des Fluiddampfbeaufschlagungsabschnittes der Fallstrecke mit Fluiddampf beaufschlagt, wodurch der Druck auf ein zweites Druckniveau (vorliegend ein Druck Pₘₐₓ von hier beispielhaft 1,3 bar absolut) ansteigt. Während des darauffolgenden, längsten Zeitabschnittes III fällt das Pulver entlang des längeren Trocknungsabschnittes der Fallstrecke, wobei während dieses weiteren freien Falls das Druckniveau wieder abgesenkt wird, hier vorliegend bis auf ein drittes Unterdruckniveau (Pₚₒₛₜ), d.h. einen Druck von absolut etwa 0,35 bar, der etwas, nämlich um die Druckdifferenz ΔP₀ oberhalb des anfänglichen, ersten Druckniveaus Pₚᵣₑ liegt. Das getrocknete Pulver wird dann ausgeschleust. Vor der erneuten Zuführung einer nächsten Pulverportion wird zunächst noch das Druckniveau bevorzugt von Pₚₒₛₜ auf

Pₚᵣₑ abgesenkt. Bei entsprechender Dimensionierung der Vakuumpumpe und der Länge der Fallstrecke ist es auch denkbar und bevorzugt dass noch während des freien Falls ein drittes Unterdruckniveau (Pₚₒₛₜ) erreicht wird, welches dem ersten Druckniveau Pₚᵣₑ abgesenkt wird.

Im Folgenenden werten alternative, nach dem Konzept der Erfindung ausgebildete Ausführungsbeispiele erläutert, wobei zur Vermeidung von Wiederholungen im wesentlichen auf Unterschiede zu den vorangehend erläuterten Ausführungsbeispielen erläutert werden. Komponenten der im Folgenden erläuterten Ausführungsbeispiele sind auch durch Komponenten der voranstehend erläuterten Ausführungsbeispiele austauschbar und umgekehrt.

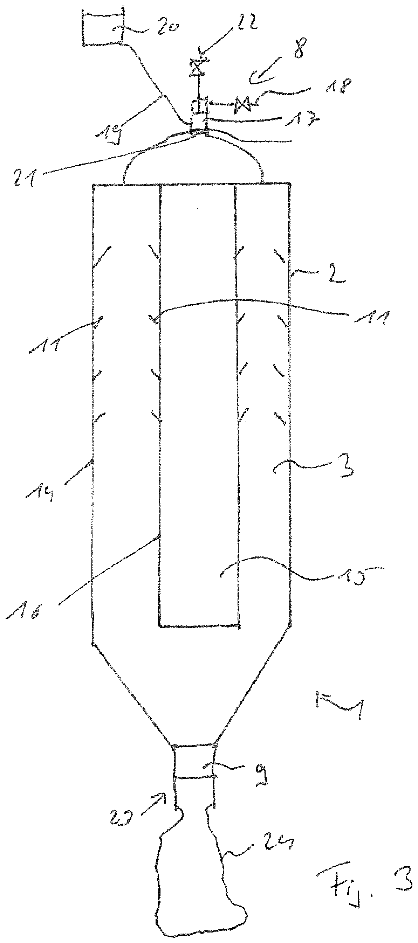

In Fig. 3 ist eine Entkeimungsvorrichtung 1 gezeigt, bei der der Behandlungsraum 3 in einer Querschnittsansicht ringförmig, vorliegend kreisringsförmig ausgebildet ist. Der Behälter 2 umfasst eine radial äußere Wand 14, die den Behälter 2 analog zu den vorstehend erläuterten Ausführungsbeispielen radial außen begrenzt. In einem zentralen Bereich umfasst bzw. beinhaltet der Behälter 2 einen, vorliegend kreiszylindrischen Zentralabschnitt 15 mit einer inneren Behälterwand 16, die den Behandlungsraum 3 radial innen begrenzt. Zu erkennen ist, dass Fluiddampfbeaufschlagungsmittel 11, vorliegend beispielhaft in Form von Ringdüsenanordnungen radial außen, d.h. sowohl im Bereich der Außenwand 14 als auch radial innen, d.h. im Bereich der Innenwand 16 des Zentralabschnitts 15 angeordnet sind, um Fluiddampf sowohl von radial außen als auch von radial innen, insbesondere schräg nach oben in den Behandlungsraum 3 einspeisen zu können, um somit für eine gleichmäßige Durchmischung mit dem zu entkeimenden Pulver während des freien Falls zu sorgen. Vorliegend reicht der Zentralabschnitt 15 bis zu einer oberen Behältergrenze, wobei in einem oberen Bereich alternativ der Behandlungsraum 3 auch in seinen Zentralbereich frei ausgestaltet sein kann.

Vorliegend ist die Einlass-Unterdruckschleuse 8 nicht als Flügelradschleuse ausgebildet, sondern als Volumen-Dosiereinrichtung, die vakuumbasiert arbeitet. Hierzu umfasst die Einlass-Unterdruckschleuse 8 eine Dosierkammer 17, die über eine Unterdruckleitung 18 mit Unterdruck beaufschlagbar ist, um somit über eine Zuführleitung 19 Pulver aus einem Pulvervorrat 20 (Pulvervorratsbehälter) ansaugen zu können. Hierbei ist die Dosierkammer 17 auslassseitig über Verschlussmittel 21 verschlossen. Nach dem Befüllen der Dosierkammer 17 wird die Dosierkammer 17 über eine Druckgasleitung 22, insbesondere mit steriler Druckluft beaufschlagt und die Dosierkammer 17 wird durch entsprechendes Verstellen der Verschlussmittel 21 in Richtung Behälter 2 bzw. Behandlungsraum 3 geöffnet und das dosierte Pulvervolumen wird in den hier lediglich beispielhaft ringförmig konturierten Behandlungsraum 3 zugeführt. Fakultative Verteilermittel sind aus Übersichtlichkeitsgründen nicht eingezeichnet. Nach Durchfallen der Fallstrecke gelangt das durch Dampfbeaufschlagung entkeimte und anschließend während des freien Falls wieder getrocknete Pulver zu einer Auslassunterdruckschleuse 9 zu einer direkt anschließenden Verpackungseinrichtung 23, mithilfe derer das Pulver schwerkraftbedingt in hier beispielhaft als Beutel ausgebildete Endverpackungen 24 verpackt werden kann.

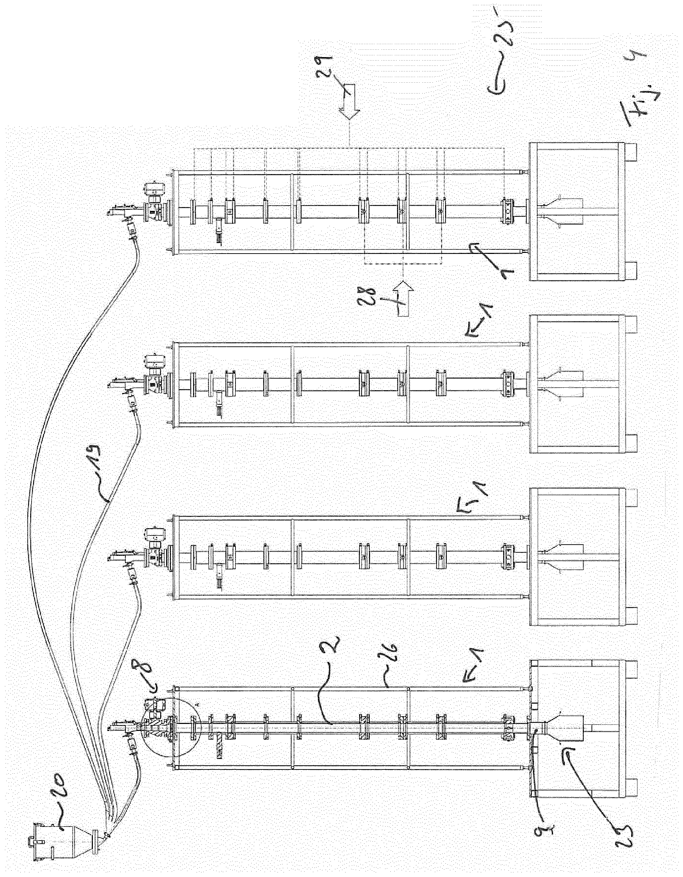

Bei dem Ausführungsbeispiel gemäß Fig. 4 handelt es sich um ein System 25 mit mehreren Entkeimungsvorrichtungen 1. Zu erkennen ist ein jeweiliger, langgestreckter Behälter 2 mit integrierter Fallstrecke 7 sowie Düsenanordnungen zum Einspeisen von Dampf. In einem unteren Bereich weist jede Entkeimungsvorrichtung 1 eine eigene Verpackungseinrichtung 23 auf.

Zu erkennen sind Halte- bzw. Stützgestelle 26, die den jeweiligen Behälter 2 tragen sowie die jeweilige Einlass-Unterdruckschleuse 8, die in der Zeichnungsebene rechts in Alleinstellung gezeigt ist. Die prinzipiell anhand von Fig. 3 gezeigte Einlass-Unterdruckschleuse 8 ist jeweils als vakuumbasierte Dosiervorrichtung ausgebildet, mit deren Hilfe über jeweils eine Zuführleitung 19 Pulver aus einem hier lediglich beispielhaft gemeinsamen Pulvervorrat 20 ansaugbar und dem jeweiligen Behälter portionsweise, volumetrisch dosiert zuführbar ist, bevorzugt durch Unterdruckbeaufschlagung und/oder mittels eines Auswurfstempels nach Öffnen von Verschlussmitteln der Dosierkammer.

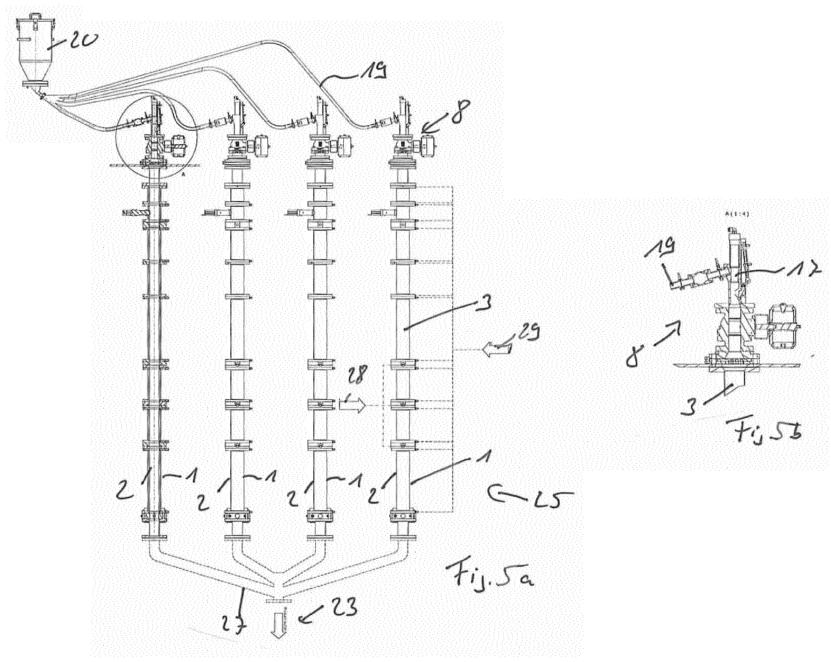

Das Ausführungsbeispiel gemäß den Fig. 5a und 5b entspricht im Wesentlichen dem Ausführungsbeispiel gemäß Fig. 4, wobei im Unterschied zu dem Ausführungsbeispiel gemäß Fig. 4 die einzelnen Entkeimungsvorrichtungen 1 bzw. deren Behälter 2 auslassseitig miteinander über Rohrleitungen 27 verbunden sind, die zu einer gemeinsamen Verpackungseinrichtung 23 oder alternativ zu einem gemeinsamen Vorratsbehälter führen. Auch bei dem Ausführungsbeispiel gemäß Fig. 5a sind die Einlass-Unterdruckschleusen als VolumenDosiereinrichtungen ausgebildet, die über Zuführleitungen 19 Pulver aus einem Pulvervorrat 20 ansaugen und dann mittels eines Stempels und/oder durch Druckgasbeaufschlagung nach Öffnen von Dosierkammerverschlussmitteln dem jeweiligen Behandlungsraum 3 zuführen.

Mit den Bezugszeichen 28 ist die Zuführung von, bevorzugt überhitzten Dampf in ein Dampfverteilungssystem gezeigt, über dass Dampf den im Behandlungsraum 3 angeordneten Auslassöffnungen, insbesondere Ringdüsenanordnung zuführbar ist. Mit dem Bezugszeichen 29 ist ein Heizmittelvorlauf, hier vorliegend ein Ölvorlauf für die Außenwandbeheizung zugeordnet.

### Bezugzeichen

- 1: Entkeimungsvorrichtung
- 2: Behälter
- 3: Behandlungsraum
- 4: Heizmittel
- 5: Vakuumpumpe
- 6: Evakuierungsmittel
- 7: Fallstrecke
- 8: Einlass-Unterdruckschleuse
- 9: Auslass-Unterdruckschleuse
- 10: Verteilmittel
- 11: Fluiddampfbeaufschlagungsmittel
- 12: Ringdüsenanordnung
- 13: Ringdüsenanordnung
- 14: äußere Behälterwand
- 15: Zentralabschnitt
- 16: innere Behälterwand
- 17: Dosierkammer
- 18: Unterdruckleitung
- 19: Zuführleitung
- 20: Pulvervorrat
- 21: Verschlussmittel
- 22: Druckgasleitung
- 23: Verpackungseinrichtung
- 24: Endverpackung
- 25: System
- 26: Haltegestell
- 27: Rohrleitungen
- 28: Pfeil (Dampfzuführung)
- 29: Pfeil (Heizmittelvorlauf)
- V: Vertikale
- Pₚᵣₑ: Unterdruckniveau bei Zuführung von Pulver (erstes Unterdruckniveau)
- Pₘₐₓ: Druckniveau unmittelbar nach Fluiddampfbeaufschlagung (zweites Unterdruckniveau)
- Pₚₒₛₜ: Unterdruckniveau am Ende des Trocknungsprozesses (drittes Unterdruckniveau)

## Patentansprüche

1. Pulverentkeimungsverfahren zum Entkeimen von Pulver, insbesondere einem pharmazeutischen Wirkstoffpulver oder einem Lebensmittelpulver, mit Fluiddampf, insbesondere Wasserdampf, wobei das Pulver in einem evakuierten Behandlungsraum (3) mit dem Fluiddampf beaufschlagt wird, wobei das Pulver während eines freien Falls entlang einer vertikalen Fallstrecke (7) mit dem Fluiddampf, insbesondere im Gegenstrom, beaufschlagt wird **dadurch gekennzeichnet,**
**dass** Evakuierungsmittel zur Bereitstellung eines Unterdrucks im Behandlungsraum (3) dauerhaft betrieben werden, sodass ständig ein Saugdruck anliegt, und der Druck nach einem Zuführen des Pulvers während des freien Falls durch Fluiddampfbeaufschlagung ansteigt.

2. Verfahren nach Anspruch 1,
**dadurch gekennzeichnet,**
**dass** Fluiddampf auf einer Pulveroberfläche kondensiert und das Pulver vor dem Abführen aus dem Behandlungsraum (3), bevorzugt während des freien Falls, wieder getrocknet wird durch Verdampfen von Kondensat von der Pulveroberfläche.

3. Verfahren nach einem der Ansprüche 1 oder 2,
**dadurch gekennzeichnet,**
**dass** das zu entkeimende Pulver bei auf einem ersten Unterdruckniveau (Pₚᵣₑ), bevorzugt zwischen 0,1mbar und 300mbar, besonders bevorzugt zwischen 0,1mbar und 100mbar, befindlichem Behandlungsraum (3) diesem zugeführt wird und während des freien Falls entlang der Fallstrecke (7) mit dem Fluiddampf beaufschlagt wird, und dass das der Druck im Behandlungsraum (3), insbesondere auf ein zweites Druckniveau (Pₘₐₓ) oberhalb von 0,5bar, bevorzugt aus einem Wertebereich zwischen 0,7bar und 4bar, bevorzugt 1bar und 3bar ansteigt und dass das Druckniveau vor dem Abführen des Pulvers aus dem Behandlungsraum (3), insbesondere noch während des freien Falls des Pulvers entlang der Fallstrecke (7), zum Trocknen des Pulvers abgesenkt wird, insbesondere auf ein, bevorzugt dem ersten Unterdruckniveau (Pₚᵣₑ) entsprechendes, drittes Unterdruckniveau (Pₚₒₛₜ), vorzugsweise aus einem Wertebereich zwischen 0,1mbar und 600mbar, bevorzugt 0,1mbar und 300mbar.

4. Verfahren nach einem der vorhergehenden Ansprüche,
**dadurch gekennzeichnet,**
**dass** zu entkeimendes Pulver über eine Einlass-Unterdruckschleuse (8), insbesondere eine Flügelradschleuse, dem Behandlungsraum (3) zugeführt und nach dem freien Fall entlang der Fallstrecke (7) und nach der währenddessen durchgeführten Fluiddampfbeaufschlagung entkeimtes Pulver über eine Auslass-Unterdruckschleuse (9), insbesondere eine Flügelradschleuse, aus dem Behandlungsraum (3) abgeführt wird.

5. Verfahren nach Anspruch 4,
**dadurch gekennzeichnet,**
**dass** zu entkeimendes Pulver ausschließlich während, oder ausschließlich nach, oder während und nach, dem Abführen von entkeimten Pulver dem Behandlungsraum (3) zugeführt wird.

6. Verfahren nach einem der vorhergehenden Ansprüche,
**dadurch gekennzeichnet,**
**dass** der Fluiddampf mit einem verglichen mit dem Druckniveau im Behandlungsraum (3) zum Zeitpunkt eines Startes der Fluiddampfbeaufschlagung erhöhten Druck, insbesondere aus einem Wertebereich zwischen 1,1bar und 6bar, weiter bevorzugt zwischen 1,1bar und 4bar, in den Behandlungsraum (3) eintritt.

7. Verfahren nach einem der vorhergehenden Ansprüche,
**dadurch gekennzeichnet,**
**dass** der Fluiddampf an mehreren vertikal beabstandeten Stellen entlang der Fallstrecke (7) beabstandeten und/oder an mehreren in Umfangsrichtung beabstandeten Stellen in den Behandlungsraum (3) eintritt.

8. Verfahren nach einem der vorhergehenden Ansprüche,
**dadurch gekennzeichnet,**
**dass** der Fluiddampf über mindestens eine sich in Umfangsrichtung erstreckende Ringdüsenanordnung (12, 13), bevorzugt über mehrere vertikal beabstandete, sich jeweils in Umfangsrichtung erstreckende Ringdüsenanordnungen (12, 13) entlang der Fallstrecke (7), in den Behandlungsraum (3) eintritt.

9. Verfahren nach einem der vorhergehenden Ansprüche,
**dadurch gekennzeichnet,**
**dass** der Fluiddampf gesättigt oder überhitzt ist und vorzugsweise bei Eintritt in den Behandlungsraum (3) eine Temperatur aus einem Wertebereich zwischen 101°C und 200°C, besonders bevorzugt zwischen 110°C und 160°C aufweist.

10. Verfahren nach einem der vorhergehenden Ansprüche,
**dadurch gekennzeichnet,**
**dass** der Behandlungsraum (3), insbesondere mittels einer Wandheizung, auf eine Temperatur aus einem Wertebereich zwischen 150°C und 400°C, bevorzugt zwischen 180°C und 300°C erwärmt wird.

11. Verfahren nach einem der vorhergehenden Ansprüche,
**dadurch gekennzeichnet,**
**dass** das Pulver, bevorzugt oberhalb und/oder in einem Anfangsbereich der Fallstrecke (7), zum Verbessern einer Pulverwolkenbildung verteilt und/oder vereinzelt und/oder verwirbelt und/oder aufgelockert wird.

## Claims

1. A powder sterilization method for sterilizing powder, in particular an active pharmaceutical ingredient powder or a food powder, using fluid vapor, in particular steam, the fluid vapor being applied to the powder in an evacuated treatment chamber (3), the fluid vapor being applied to the powder, in particular in countercurrent, during a free fall of the powder along a drop section (7),
**characterized in that**
evacuation means for providing a negative pressure in the treatment chamber (3) are operated in a permanent and continuous manner to provide a negative pressure in the treatment chamber, a suction pressure thus being permanently applied and the application of the fluid vapor causing the pressure to rise during the free fall after the powder has been supplied.

2. The method according to claim 1,
**characterized in that**
the fluid vapor condenses on a powder surface and the powder is dried by evaporating condensate from the powder surface, preferably during the free fall, before discharging the powder from the treatment chamber (3).

3. The method according to claim 1 or 2,
**characterized in that**
the powder to be sterilized is supplied to the treatment chamber (3) when the treatment chamber (3) is at a first negative pressure level (Pₚᵣₑ), preferably between 0.1 mbar and 300 mbar, particularly preferably between 0.1 mbar and 100 mbar, and the fluid vapor is applied to said powder during the free fall along the drop section (7), and that the pressure in the treatment chamber (3) rises, in particular to a second pressure level (Pₘₐₓ) above 0.5 bar, preferably from a range between 0.7 bar and 4 bar, preferably 1 bar and 3 bar, and that the pressure level is reduced for drying the powder before the powder is discharged from the treatment chamber (3), in particular while the powder is still falling freely along the drop section (7), in particular to a third negative pressure value (Pₚₒₛₜ), preferably corresponding to the first negative pressure level (Pₚᵣₑ), preferably from a range between 0.1 mbar an 600 mbar, preferably 0.1 mbar and 300 mbar.

4. The method according to any one of the preceding claims, **characterized in that**
powder to be sterilized is supplied to the treatment chamber (3) via an inlet negative pressure lock (8), in particular an impeller lock, and that sterilized powder is discharged from the treatment chamber (3) via an outlet negative pressure lock (9), in particular an impeller lock, after the free fall along the drop section (7) and after the application of fluid vapor carried out during the free fall.

5. The method according to claim 4,
**characterized in that**
powder to be sterilized is supplied to the treatment chamber (3) solely during, or solely after, or during and after the discharging of sterilized powder.

6. The method according to any one of the preceding claims, **characterized in that**
the fluid vapor enters the treatment chamber (3) at an increased pressure, in particular from a range between 1.1 bar and 6 bar, more preferably between 1.1 bar and 4 bar, compared to the pressure level in the treatment chamber (3) at the time the fluid vapor application starts.

7. The method according to any one of the preceding claims, **characterized in that**
the fluid vapor enters the treatment chamber (3) at several points spaced apart in the vertical direction and/or at several points spaced apart in the circumferential direction.

8. The method according to any one of the preceding claims, **characterized in that**
the fluid vapor enters the treatment chamber (3) via at least one ring nozzle assembly (12, 13) extending in the circumferential direction, preferably via several ring nozzle assemblies (12, 13) spaced apart in a vertical manner and each extending in the circumferential direction along the drop section (7).

9. The method according to any one of the preceding claims, **characterized in that**
the fluid vapor is saturated or superheated and preferably has a temperature from a range between 101 °C and 200 °C, particularly preferably between 110 °C and 160 °C, when it enters the treatment chamber (3).

10. The method according to any one of the preceding claims, **characterized in that**
the treatment chamber (3) is heated to a temperature from a range between 150 °C and 400 °C, preferably between 180 °C and 300 °C, in particular by means of a wall heating device.

11. The method according to any one of the preceding claims, **characterized in that**
the powder is dispersed and/or separated and/or swirled and/or loosened, preferably above and/or in an initial area of the drop section (7), in order to improve powder cloud formation.

## Revendications

1. Procédé de stérilisation de poudre pour stériliser de la poudre, notamment une poudre de principe actif pharmaceutique ou une poudre alimentaire, en utilisant de la vapeur de fluide, notamment de la vapeur d'eau, la poudre étant alimentée en vapeur de fluide dans une chambre de traitement (3) évacuée, la poudre étant alimentée, notamment dans le contre-courant, en vapeur de fluide le long d'un trajet (7) vertical de chute pendant une chute libre,
**caractérisé en ce que**
des moyens d'évacuation pour fournir une pression négative dans la chambre de traitement (3) fonctionnent en permanence de sorte qu'une pression d'aspiration est toujours appliquée et la pression augmente à cause d'une alimentation en vapeur de fluide pendant la chute libre après que la poudre a été fournie.

2. Procédé selon la revendication 1,
**caractérisé en ce que**
de la vapeur de fluide condense sur une surface de la poudre et la poudre est séchée de nouveau en évaporant du condensat de la surface de la poudre avant de l'évacuation de la chambre de traitement (3), notamment pendant la chute libre.

3. Procédé selon la revendication 1 ou 2,
**caractérisé en ce que**
si une chambre de traitement (3) se trouve à un premier niveau (Pₚᵣₑ) de pression négative, de préférence entre 0,1 mbar et 300 mbar, de préférence particulière entre 0,1 mbar et 100 mbar, la poudre à stériliser est fournie à ladite chambre de traitement (3) et alimentée en vapeur de fluide le long du trajet (7) de chute pendant la chute libre, et **en ce que** la pression dans la chambre de traitement (3) augmente notamment à un deuxième niveau de pression (Pₘₐₓ) qui est supérieur à 0,5 bar, de préférence d'une plage de valeurs entre 0,7 bar et 4 bar, de préférence 1 bar et 3 bar, et **en ce que**, avant de l'évacuation de la poudre de la chambre de traitement (3), notamment encore pendant la chute libre de la poudre le long du trajet (7) de chute, le niveau de pression est réduit notamment à un troisième niveau (Pₚₒₛₜ) de pression négative qui, de préférence, correspond au premier niveau (Pₚᵣₑ) de pression négative et qui est, de préférence, d'une plage de valeurs entre 0,1 mbar et 600 mbar, préférentiellement entre 0,1 mbar et 300 mbar, pour sécher la poudre.

4. Procédé selon l'une quelconque des revendications précédentes, **caractérisé en ce que**
de la poudre à stériliser est fournie à la chambre de traitement (3) via un sas (8) de pression négative d'entrée, notamment un sas à hélice, et de la poudre stérilisée est évacuée de la chambre de traitement (3) via un sas (9) de pression négative de sortie, notamment un sas à hélice, après la chute libre le long du trajet (7) de chute et après l'alimentation en vapeur de fluide effectuée pendant la chute libre.

5. Procédé selon la revendication 4,
**caractérisé en ce que**
de la poudre à stériliser est fournie à la chambre de traitement (3) uniquement pendant, ou uniquement après, ou pendant et après l'évacuation de poudre stérilisée.

6. Procédé selon l'une quelconque des revendications précédentes, **caractérisé en ce que**
la vapeur de fluide entre dans la chambre de traitement (3) avec une pression qui est, comparée avec le niveau de pression dans la chambre de traitement (3) au moment où l'alimentation en vapeur de fluide commence, augmentée, notamment d'une plage de valeurs entre 1,1 bar et 6 bar, encore plus de préférence entre 1,1 bar et 4 bar.

7. Procédé selon l'une quelconque des revendications précédentes, **caractérisé en ce que**
la vapeur de fluide entre dans la chambre de traitement (3) à plusieurs endroits espacés verticalement le long du trajet (7) de chute et/ou à plusieurs endroits espacés dans une direction circonférentielle.

8. Procédé selon l'une quelconque des revendications précédentes, **caractérisé en ce que**
la vapeur de fluide entre dans la chambre de traitement (3) via au moins un ensemble (12, 13) de tuyères annulaires s'étendant dans une direction circonférentielle, de préférence via plusieurs ensembles (12, 13) de tuyères annulaires le long du trajet (7) de chute, lesdits plusieurs ensembles (12, 13) de tuyères annulaires étant verticalement espacés et s'étendant chacun dans une direction circonférentielle.

9. Procédé selon l'une quelconque des revendications précédentes, **caractérisé en ce que**
la vapeur de fluide est saturée ou surchauffée et, de préférence, a une température d'une plage de valeurs entre 101 °C et 200°C, de préférence particulière entre 110°C et 160°C, lorsqu'elle entre dans la chambre de traitement (3).

10. Procédé selon l'une quelconque des revendications précédentes, **caractérisé en ce que**
la chambre de traitement (3) est chauffée à une température d'une plage de valeurs entre 150°C et 400°C, de préférence entre 180°C et 300°C, notamment au moyen d'un chauffage mural.

11. Procédé selon l'une quelconque des revendications précédentes, **caractérisé en ce que**
la poudre est répartie et/ou séparée et/ou tourbillonnée et/ou aérée, de préférence au-dessus et/ou dans une zone initiale du trajet (7) de chute, pour améliorer une formation de nuages de poudre.
